# EUROPEAN PATENT APPLICATION

(11) **EP 3 808 358 A1**
(43) Date of publication of application: **21.04.2021**
(21) Application number: 19203965.9
(22) Date of filing: 17.10.2019
(51) Int. Cl.: A61K 35/741, A61P 1/00, A61P 3/04, A61P 3/10, A61P 31/00, A61P 37/04, A61P 43/00

(54) **MICROBIAL COMPOSITIONS, STRAINS AND METHODS**

(71) Applicant: University College Cork-National University of Ireland, Cork, Cork (IE)
(72) Inventor: O'TOOLE, Paul, Cork (IE); JEFFERY, Ian, Cork (IE); GHOSH, Tarini, Cork (IE); TAN, Huizi, Cork (IE); PEREZ, Marta, Cork (IE); NTEMIRI, Alexandrea, Cork (IE)
(74) Representative: Murgitroyd & Company

(57) **Abstract**

A probiotic composition is provided comprising at least two isolated bacterial species or comprising a purified bacterial preparation comprising at least two bacterial species, wherein the at least two bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* Also provided are specific isolated bacterial strains and use of the composition for treating frailty and/or inflammation related to aging in elderly subjects.

## Description

### Field of the Invention

The present invention relates to microbial compositions and strains which may be used to treat conditions where dysbiosis is implicated and, in particular, may be used for delaying frailty and treating inflammation associated with ageing. Also provided is a method of preparing a modified artificial bacterial consortium for use in treating a condition where dysbiosis is implicated.

### Background of the Invention

Alterations in the gut microbiome have been reported for many diseases. Identifying the specificity of these alterations is necessary for developing microbiome-based diagnostic and therapeutic strategies. Meta-analyses across cases and controls from different locations have identified microbiome alterations common to multiple diseases, as well as disease-specific alterations. While certain diseases like colorectal cancer (CRC) are characterised by an increase (or gain) of pathobionts (such as *Fusobacterium, Porphyromonas, Parvimonas*), the onset of others like Inflammatory Bowel Disease (IBD) is associated with the depletion of specific taxa (e.g. *Roseburia, Faecalibacterium*). In contrast, diarrhoeal diseases are accompanied by both an increase of pathobionts (specifically *Enterobacteriaceae*) as well as lower abundance of commensal taxa. While species like *Faecalibacterium prausnitzii* and *Roseburia* have been shown to be reduced in inflammatory and metabolic disorders like Type 2 diabetes, *Barnesiella intestinihominis* has been indicated to prevent onset of colorectal cancer and *Clostridium difficile* infections (two disorders that are predominant in the elderly). Similarly, species belonging to *Coprococcus* genus have been indicated to be associated with reduced depression.

Frailty refers to a generic failure of multiple systems. Tools for measuring frailty include the Functional Independence Measure (FIM) and the Barthel Index. It has been shown that frail elderly subjects have a microbiota profile characterised by low diversity compared to healthy elderly subjects. In particular, a microbiota profile characterised by low diversity is associated with subjects that live in long-term residential care and who consume a diet low in fibre and enriched in saturated fat. Reducing the prevalence or severity of frailty in the elderly would be beneficial for decreasing the risk of serious injury or illness in elderly subjects, for example as a result of a fall or otherwise. Treatments to date include exercise and nutritional interventions.

Ageing has been associated with specific changes in the gut microbiome, which include a loss of specific bacterial species. This could be a consequence of specific dietary changes, intake of medications and an overall decline of immune status with age and is likely to drive the ageing gut microbiome to an increasingly disease susceptible state. Ageing-associated microbiome alterations have been identified in the ELDERMET cohort, reinforced by lower complexity dietary intake and poly-pharmacy (Borrel G., et al. Genomics and metagenomics of trimethylamine-utilizing Archaea in the human gut microbiome. ISME J. 2017 Sep; 11(9): 2059-2074). Human aging is characterised by a chronic, low-grade inflammation and this phenomenon has been termed "inflammaging". Inflammaging is a highly significant risk factor for mortality in the elderly.

Interventions targeting the gut microbiota, such as the use of live biotherapeutic consortia, provide a promising approach to treat diseases and conditions associated with gut microbiota alterations. Faecal microbiota transplantation (FMT) has been shown to be highly effective for curing *Clostridium difficile*-associated diarrhoea (CDAD), but requires extensive screening of donors to eliminate the chance of administering infectious agents. Furthermore, the biological impact of transplanting an at-risk patient with an essentially uncharacterised inoculum is unknown, especially in relation to long-term effects. It has been shown that a relatively simple cocktail of 33 cultured and lab-purified isolates is effective against CDAD. Methods to maintain or restore gut integrity and/or a youthful microbiota are also considered to hold promise for reducing age-related inflammation (Franceschi and Campisis, Chronic Inflammation (Inflammaging) and Its Potential Contribution to Age-Associated Diseases. J. Gerontol. A. Biol. Sci. Med. Sci. 2014, June; 69(S1):S4-S9).

Microbiome reconstruction strategies can occur through diverse approaches, but the two simplest and most direct types are diet-based and microbe-based. Dietary formulations, while demonstrating reasonably high efficiency in the infants/children, have been shown to be inefficient and subject to patient compliance in older subjects. Microbe-based strategies include faecal microbiome transplantation and live biotherapeutics. Currently available probiotic supplements are mostly based on *Lactobacillus,* or *Bifidobacterium* species. Although these supplements may have high efficacy for the infant, children and young adult populations, the associations of these species with health have not been convincingly demonstrated for the elderly. Elderly people experience distinct changes in physiology and dietary patterns, accompanied by characteristics that are distinct from those in the younger populations. Appropriate microbiome-based therapeutic strategies that facilitate the retention of a health-associated gut microbiome in the elderly and prevent or delay the onset of frailty and age-related inflammation would therefore be of benefit. In particular, the provision of a therapeutic microbiome restoration consortium for use in the elderly population containing a rationally selected set of microbial species whose loss in the elderly is associated with frailty onset and age-related inflammation may be advantageous in delaying or reducing frailty and age-related inflammation in the elderly.

### Summary of the Invention

The present invention provides a composition comprising one or more isolated bacterial species or comprising a purified bacterial preparation comprising one or more bacterial species, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

The composition allows for the maintenance or restoration of a healthy microbiota in the gastrointestinal tract of a mammalian subject. The composition may be used for microbiome reconstruction or remediation. In particular, the composition may be used as therapeutic agents in conditions where dysbiosis (deviation from typical microbiome composition associated with health) is implicated, such as frailty and inflammation associated with ageing. The composition may restore a health-associated mammalian bacterial intestinal microbiota. In particular, the composition may increase the diversity and/or richness of a subject's microbiota profile and reduce the risk of adverse health effects, for example, frailty and inflammation associated with ageing. The composition may restore specific bacterial species and beneficial metabolic processes which have been lost from the microbiome due to ageing. The required species may be identified by comparing the subject's faecal microbiota with that of one or more healthy subjects. In particular, the composition allows the rectification of the gut microbiota of frail elderly subjects, for example, using live biotherapeutic units having a defined microbial configuration. This avoids any risk associated with the use of an uncharacterised inoculum.

The invention further extends to a pharmaceutical or probiotic composition comprising the above composition and a pharmaceutically acceptable carrier.

Also provided is a composition as described above for use as a medicament.

According to a further aspect of the present invention, there is provided a composition for use in treating or delaying onset of frailty in a subject in need thereof, the composition comprising one or more isolated bacterial species or comprising a purified bacterial preparation comprising one or more bacterial species, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

Also provided is a method for treating or delaying onset of frailty in a subject in need thereof, the method comprising administering a composition comprising one or more isolated bacterial species or comprising a purified bacterial preparation comprising one or more bacterial species to the subject, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

Also provided is use of a composition comprising one or more isolated bacterial species or comprising a purified bacterial preparation comprising one or more bacterial species in the preparation of a medicament for treating or delaying onset of frailty in a subject in need thereof, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

According to a further aspect of the present invention, there is provided a composition for use in treating or delaying onset of inflammation related to aging (inflammaging) in a subject in need thereof, the composition comprising one or more isolated bacterial species or comprising a purified bacterial preparation comprising one or more bacterial species, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

Also provided is a method for treating or delaying onset of inflammation related to aging (inflammaging) in a subject in need thereof, the method comprising administering a composition comprising one or more isolated bacterial species or comprising a purified bacterial preparation comprising one or more bacterial species to the subject, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

Also provided is use of a composition comprising one or more isolated bacterial species or comprising a purified bacterial preparation comprising one or more bacterial species in the preparation of a medicament for treating or delaying onset of inflammation related to aging (inflammaging) in a subject in need thereof, wherein the one or more bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.* The composition or the purified bacterial preparation may comprise at least two of the one or more bacterial species.

In certain embodiments of the above aspects, the composition comprises, consists essentially of or consists of two or more, three or more, four or more, five or more, six or more, or all seven species selected from the group consisting of *Coprococcus catus, Eubacterium rectale, Alistipes putredinis, Barnesiella intestinihominis, Roseburia hominis, Dorea longicatena* and *Faecalibacterium prausnitzii.* In certain embodiments of the above aspects, the composition comprises, consists essentially of or consists of at least two, at least three, at least four, at least five, at least six or all seven species selected from the group consisting of *Coprococcus catus, Eubacterium rectale, Alistipes putredinis, Barnesiella intestinihominis, Roseburia hominis, Dorea longicatena* and *Faecalibacterium prausnitzii.* In certain embodiments of the above aspects, the composition comprises, consists essentially of or consists of two, three, four, five, six or all seven species selected from the group consisting of *Coprococcus catus, Eubacterium rectale, Alistipes putredinis, Barnesiella intestinihominis, Roseburia hominis, Dorea longicatena* and *Faecalibacterium prausnitzii.*

In certain embodiments of the above aspects, the one or more bacterial species comprise strains selected from the group consisting of *Coprococcus catus* (MCC394) deposited under NCIMB 43487, *Eubacterium rectale* (MCC552) deposited under NCIMB 43489, *Alistipes putredinis* (MCC001) deposited under NCIMB 43485, *Barnesiella intestinihominis* (MCC256) deposited under NCIMB 43486, *Roseburia hominis* (MCC694) deposited under NCIMB 43491, *Dorea longicatena* (MCC451) deposited under NCIMB 43488 and *Faecalibacterium prausnitzii* (MCC585) deposited under NCIMB 43490.

In certain embodiments of the above aspects, the one or more bacterial species comprise strains selected from the group consisting of *Coprococcus catus* comprising, consisting essentially of or consisting of amino sequence SEQ ID No. 1 or a variant sequence having at least 90% sequence identity thereto, *Eubacterium rectale* comprising, consisting essentially of or consisting of amino sequence SEQ ID No. 2 or a variant sequence having at least 90% sequence identity thereto, *Alistipes putredinis* comprising, consisting essentially of or consisting of amino sequence SEQ ID No. 3 or a variant sequence having at least 90% sequence identity thereto, *Barnesiella intestinihominis* comprising, consisting essentially of or consisting of amino sequence SEQ ID No. 4 or a variant sequence having at least 90% sequence identity thereto, *Roseburia hominis* comprising, consisting essentially of or consisting of amino sequence SEQ ID No. 5 or a variant sequence having at least 90% sequence identity thereto, *Dorea longicatena* comprising, consisting essentially of or consisting of amino sequence SEQ ID No. 6 or a variant sequence having at least 90% sequence identity thereto and *Faecalibacterium prausnitzii* comprising, consisting essentially of or consisting of amino sequence SEQ ID No. 7 or a variant sequence having at least 90% sequence identity thereto.

In certain embodiments of the above aspects, the composition comprises, consists essentially of or consists of the genera shown in Table 1 (the Microbiome Culture Collection 100 (MCC100)). In certain embodiments of the above aspects, the one or more bacterial species comprises one or more strains of a single species of bacteria, e.g. one or more strains of *Coprococcus catus, Eubacterium rectale, Alistipes putredinis, Barnesiella intestinihominis, Roseburia hominis, Dorea longicatena* and/or *Faecalibacterium prausnitzii.*

According to a further aspect of the present invention, there is provided an isolated bacterial strain selected from the group consisting of *Coprococcus catus* (MCC394) deposited under NCIMB 43487, *Eubacterium rectale* (MCC552) deposited under NCIMB 43489, *Alistipes putredinis* (MCC001) deposited under NCIMB 43485, *Barnesiella intestinihominis* (MCC256) deposited under NCIMB 43486, *Roseburia hominis* (MCC694) deposited under NCIMB 43491, *Dorea longicatena* (MCC451) deposited under NCIMB 43488 and *Faecalibacterium prausnitzii* (MCC585) deposited under NCIMB 43490.

According to a further aspect of the present invention, there is provided an isolated bacterial strain selected from the group consisting of *Coprococcus catus* comprising, consisting essentially of or consisting of amino sequence SEQ ID No. 1 or a variant sequence having at least 90% sequence identity thereto, *Eubacterium rectale* comprising, consisting essentially of or consisting of amino sequence SEQ ID No. 2 or a variant sequence having at least 90% sequence identity thereto, *Alistipes putredinis* comprising, consisting essentially of or consisting of amino sequence SEQ ID No. 3 or a variant sequence having at least 90% sequence identity thereto, *Barnesiella intestinihominis* comprising, consisting essentially of or consisting of amino sequence SEQ ID No. 4 or a variant sequence having at least 90% sequence identity thereto, *Roseburia hominis* comprising, consisting essentially of or consisting of amino sequence SEQ ID No. 5 or a variant sequence having at least 90% sequence identity thereto, *Dorea longicatena* comprising, consisting essentially of or consisting of amino sequence SEQ ID No. 6 or a variant sequence having at least 90% sequence identity thereto and *Faecalibacterium prausnitzii* comprising, consisting essentially of or consisting of amino sequence SEQ ID No. 7 or a variant sequence having at least 90% sequence identity thereto.

The variant amino acid sequence may have at least 91%, 92/%, 93%, 94%, 95%, 96%, 97%, 98% or 99% sequence identity with the amino acid sequence in question. The variant amino acid sequence may have at least 95% sequence identity with the amino acid sequence in question. The variant amino acid sequence may have at least 97% sequence identity with the amino acid sequence in question. In a particular embodiment, the variant amino acid sequence may have at least 97% sequence identity with the amino acid sequence in question for a query coverage of greater than 90%. The variant amino acid sequence may have at least 99% sequence identity with the amino acid sequence in question. The variant amino acid sequence may differ from the amino acid sequence on which it is based due to the presence of one or more conservative amino acid substitutions. Typically, the variant amino acid sequence differs from the amino acid sequence on which it is based due to the presence of less than 5, 4, 3, 2 or 1 conservative amino acid substitutions. The only differences between the variant amino acid sequence and the amino acid sequence on which it is based may be conservative amino acid substitutions, that is, no non-conservative amino acid residue alterations are present. In certain embodiments, the variant amino acid sequence may include one or more, preferably less than 3 and more preferably less than 2, truncations, substitutions, deletions or insertions.

Typically, the bacterial strain comprising the variant amino acid sequence as above retains the ability to restore or maintain a healthy mammalian bacterial intestinal microbiota, for example, to treat frailty or age-related inflammation. Typically, the bacterial strain comprising the variant amino acid sequence has the same or improved activity when compared to the bacterial strain comprising the unmodified amino acid sequence, i.e. the changes to the amino acid sequences do not result in any undesirable loss of activity.

In certain embodiments of the above aspects, the *Coprococcus catus* strain comprises SEQ ID No. 1, the *Eubacterium rectale* strain comprises SEQ ID No. 2, the *Alistipes putredinis* strain comprises SEQ ID No. 3, the *Barnesiella* intestinihominis strain comprises SEQ ID No. 4, the *Roseburia hominis* strain comprises SEQ ID No. 5, the *Dorea longicatena* strain comprises SEQ ID No. 6 and/or the *Faecalibacterium prausnitzii* strain comprises SEQ ID No. 7.

In certain embodiments of the above aspects, the *Coprococcus catus* strain comprises a 16S rDNA comprising SEQ ID No. 15, the *Eubacterium rectale* strain comprises a 16S rDNA comprising SEQ ID No. 16, the *Alistipes putredinis* strain comprises a 16S rDNA comprising SEQ ID No. 17, the *Barnesiella intestinihominis* strain comprises a 16S rDNA comprising SEQ ID No. 18, the *Roseburia hominis* strain comprises a 16S rDNA comprising SEQ ID No. 19, the *Dorea longicatena* strain comprises a 16S rDNA comprising SEQ ID No. 20 and/or the *Faecalibacterium prausnitzii* strain comprises a 16S rDNA comprising SEQ ID No. 21.

In certain embodiments, the *Coprococcus catus* bacterial strain comprises a 16S rDNA comprising a sequence at least 99.5% identical to SEQ ID NO: 15. In certain embodiments, the *Coprococcus catus* bacterial strain comprises a 16S rDNA comprising a sequence at least 99.6, 99.7, 99.8 or 99.9% identical to SEQ ID NO: 15. In certain embodiments, the *Eubacterium rectale* bacterial strain comprises a 16S rDNA comprising a sequence at least 99.5% identical to SEQ ID NO: 16. In certain embodiments, the *Eubacterium rectale* bacterial strain comprises a 16S rDNA comprising a sequence at least 99.6, 99.7, 99.8 or 99.9% identical to SEQ ID NO: 16. In certain embodiments, the *Barnesiella intestinihominis* bacterial strain comprises a 16S rDNA comprising a sequence at least 99% identical to SEQ ID NO: 18. In certain embodiments, the *Barnesiella intestinihominis* bacterial strain comprises a 16S rDNA comprising a sequence at least 99.1, 99.2, 99.3, 99.4, 99.5, 99.6, 99.7, 99.8 or 99.9% identical to SEQ ID NO: 18. In certain embodiments, the *Faecalibacterium prausnitzii* bacterial strain comprises a 16S rDNA comprising a sequence at least 99.5% identical to SEQ ID NO: 21. In certain embodiments, the *Faecalibacterium prausnitzii* bacterial strain comprises a 16S rDNA comprising a sequence at least 99.6, 99.7, 99.8 or 99.9% identical to SEQ ID NO: 21.

The invention further extends to isolated nucleic acid molecules encoding for the bacterial strain of the invention. The isolated nucleic acid molecule may be selected from the group consisting of SEQ ID No. 8 (*Coprococcus catus*), SEQ ID No. 9 (*Eubacterium rectale*), SEQ ID No. 10 (*Alistipes putredinis*), SEQ ID No. 11 (*Barnesiella intestinihominis*), SEQ ID No. 12 (*Roseburia hominis*), SEQ ID No. 13 (*Dorea longicatena*), and SEQ ID No. 14 (*Faecalibacterium prausnitzii*). The nucleic acid molecules may encode the amino acid sequences which make up the bacterial strains as described above.

The invention extends to the isolated strain of the invention for use as a medicament and to pharmaceutical, biotherapeutic or probiotic compositions comprising same. In particular, the invention extends to the isolated strain of the invention for use in treating or delaying onset of frailty or age-related inflammation in a subject in need thereof, as described above. The invention provides a method for treating or delaying onset of frailty or age-related inflammation in a subject in need thereof comprising administering the isolated strain of the invention to the subject. The invention further provides for use of the strain of the invention in the preparation of a medicament for treating or delaying onset of frailty or age-related inflammation in a subject in need thereof.

The composition or one or more of the isolated strains of the invention may be provided for use as a live biotherapeutic. As such, the invention extends to a live biotherapeutic comprising the composition or one or more of the isolated species of the invention.

The present invention further extends to an isolated polypeptide, or a fragment thereof, obtainable by culturing, or derived from, one or more of the strains of the invention. In certain embodiments of the above aspects, a product of the one or more strains may be provided in place of the one or more strains, for example, for use in treating or delaying frailty or age-related inflammation. The product may comprise a supernatant of the strain or a purified excretory product, for example, obtained by fermentation of the strain.

In certain embodiments of the above aspects, the subject is an elderly subject. In particular, the subject may be 50 years or older, 55 years or older, 60 years or older, 65 years or older, 70 years or older, 75 years or older, 80 years or older, 85 years or older or 90 years or older. In certain embodiments, the subject is 64 years or older. In certain embodiments, the subject is 60 years or older. In certain embodiments, the subject is suffering from, or at risk from suffering from, frailty or age-related inflammation. The subject may have been diagnosed as being deficient in one or more strains that are normally present in the gut of a healthy subject, e.g. one or more strains of the invention. The composition may comprise the one or more strains identified as being decreased in the subject.

In certain embodiments of the above aspects, the composition or the one or more strains is formulated for administration orally or is administered orally, for example, as capsules, tablets, powders, granules, microparticles or nanoparticles. In certain embodiments of the above aspects, the composition or the one or more strains is formulated as a probiotic. In certain embodiments of the above aspects, the composition or the one or more strains is formulated as a live biotherapeutic product. The composition may be provided as a food, as a drink or as a food supplement. The composition or the one or more strains may be configured for targeted release of the one or more strains in the intestine of the subject. In certain embodiments of the above aspects, the composition or the one or more strains is formulated for administration by, or is administered rectally, for example as a suppository. In certain embodiments of the above aspects, the composition or the one or more strains is formulated for administration by, or is administered by, techniques used for faecal microbiota transplantation (FMT), such as colonoscopy or nasojejunal gastroscopy.

According to a further aspect of the present invention, there is provided a method of preparing a modified artificial bacterial consortium for use in treating a condition where dysbiosis is implicated, the method comprising:
- comparing normal faecal microbiota of one or more healthy subjects with faecal microbiota of a subject to be treated to identify alterations in the microbiota of the subject with the condition; and
- adjusting the composition and proportional abundance of bacterial strains in an original artificial bacterial consortium to provide a modified artificial bacterial consortium that rectifies the identified alterations.

The method of the present invention provides an infinitely customisable approach and can thus be used for personalised microbiome therapy, whereby a modified artificial bacterial consortium or inoculum designed to alter the microbiota composition of individual subjects can be prepared based on profiling their microbiome composition, for example, in advance of receiving therapy.

In certain embodiments, the modified artificial bacterial consortium comprises a single strain from the original artificial bacterial consortium. However, generally the modified artificial bacterial consortium comprises more than one, and generally several, strains, for example, two, three, four, five, six, seven, eight, nine, ten, fifteen, twenty, twenty-five, thirty or more strains.

In certain embodiments, the alterations in the microbiota of the subject with the condition comprises a microbiota deficit. The composition of the bacterial strains in the original artificial bacterial consortium may be adjusted by selecting one or more bacterial strains from the original artificial bacterial consortium. The proportional abundance of the bacterial strains in the original artificial bacterial consortium may be adjusted by increasing the amount of some strains present while decreasing the amount of other strains present to provide the modified artificial bacterial consortium suitable for treating the condition in question.

The normal faecal microbiota may be derived from one or more samples obtained from one or more healthy subjects, that is, subjects who are not suffering from frailty or a disease. The healthy subjects may be of a similar age as the subject to be treated.

In certain embodiments of the above aspect, the original artificial bacterial consortium comprises, consists essentially of or consists of the genera shown in Table 1 (the Microbiome Culture Collection 100 (MCC100)).

In certain embodiments of the above aspect, the original artificial bacterial consortium is prepared by a method comprising identifying strains safe for use in the gut by sequencing the strains and testing the strains for antibiotic resistance.

In certain embodiments of the above aspect, the method includes a step of formulating the modified artificial bacterial consortium for administration to the subject to be treated.

In certain embodiments of the above aspect, the method includes a step of administering the modified artificial bacterial consortium to the subject to be treated.

According to a further aspect of the present invention, there is provided a modified artificial bacterial consortium prepared using the method described above.

Also provided is a modified artificial bacterial consortium for use in treating a condition where dysbiosis is implicated. A method for treating a condition where dysbiosis is implicated comprising administering a modified artificial bacterial consortium to a subject in need thereof and use of a modified artificial bacterial consortium in the preparation of a medicament for treating a condition where dysbiosis is implicated are also provided. The modified artificial bacterial consortium may be prepared using the method described above.

In certain embodiments of the above aspects, the condition where dysbiosis is implicated is frailty in the elderly (which may be associated with loss of microbiota diversity and short-chain fatty-acid production). In certain embodiments of the above aspects, the condition where dysbiosis is implicated is inflammation associated with aging, i.e. inflammation in the elderly. In certain embodiments of the above aspects, the condition where dysbiosis is implicated is aging. In certain embodiments of the above aspects, the condition where dysbiosis is implicated comprises infectious diseases. In certain embodiments of the above aspects, the condition where dysbiosis is implicated comprises inflammatory diseases. In certain embodiments of the above aspects, the condition where dysbiosis is implicated is cancer. In certain embodiments of the above aspects, the condition where dysbiosis is implicated is selected from the group consisting of *Clostridium difficile*-associated diarrhoea (CDAD, which may be associated with loss of microbiota diversity), colorectal cancer (which may be associated with outgrowth of several pathogen clusters and other co-abundance groups), autism (which may be associated with loss of Bacteroides subtypes and outgrowth of pathobionts), Irritable Bowel Syndrome (which may be associated with increased ratio of phylum Firmicutes to phylum Bacteroidetes, and subtypes thereof), Inflammatory Bowel Disease (which may be associated with loss of microbiota diversity and extinction of antiinflammatory taxa including *Roseburia, Eubacterium* and *Faecalibacterium*) and type 2 diabetes (which may be associated with outgrowth of pathobionts and loss of taxa that signal to the liver).

In certain embodiments of the above aspects, the modified artificial bacterial consortium is formulated as a live biotherapeutic product. In certain embodiments of the above aspects, the modified artificial bacterial consortium is formulated for administration by, or is administered by, techniques used for faecal microbiota transplantation (FMT), such as colonoscopy or nasojejunal gastroscopy. In certain embodiments, the modified artificial bacterial consortium is formulated for administration orally or is administered orally, for example, as capsules. In certain embodiments, the modified artificial bacterial consortium is formulated for administration rectally or is administered rectally.

The use of an artificial stool for treatment of conditions such as CDAD offers improvements in safety, reproducibility, quality, consistency, security of supply, speed and cost.

### Brief Description of the Figures

The invention will be more clearly understood from the following description of some embodiments thereof, given by way of example only, with reference to the following figures in which:

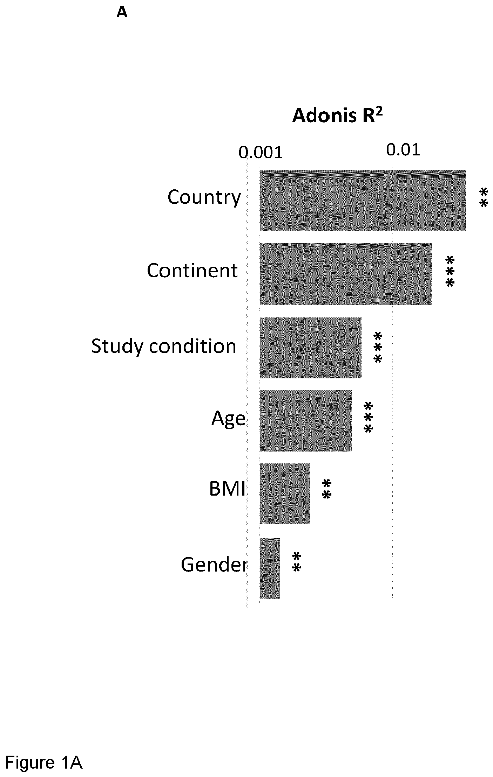

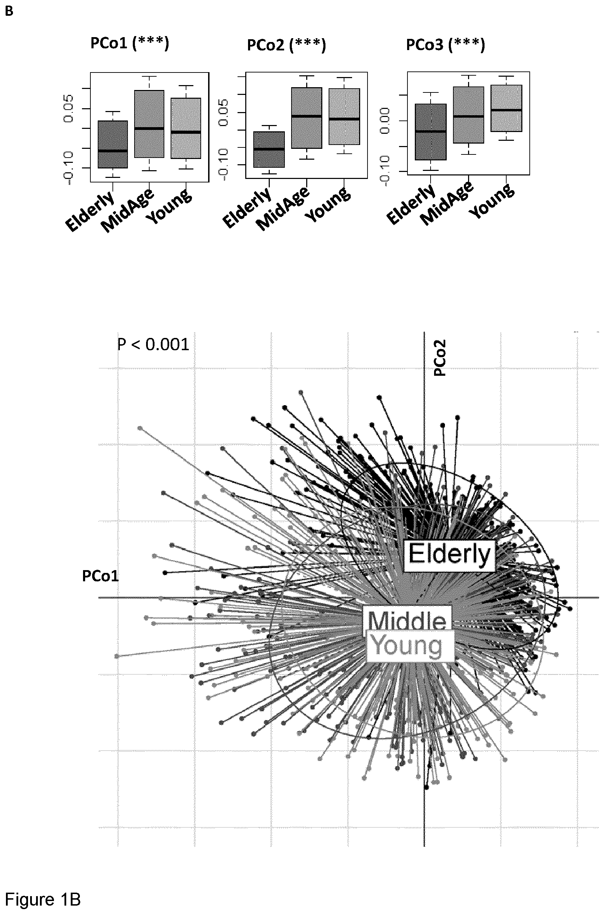

Figure 1. Age influences microbiome composition as well as microbiome-disease signatures. A. Bar plots showing the effect (denoted by R² values computed using PERMANOVA) of host factors with microbiome composition in the ExperimentHub repository. Only metadata available for at least 30% of the samples are shown. The p-values for the significance of association are also indicated as ****: P < 0.0001; ***: P < 0.001, **: P < 0.01, *: P < 0.05. B. Principal Co-ordinate Analysis (PCoA) plots of the species profiles of the 'control' samples grouped into three age ranges, Young (20-40 years), Middle (40-60 years) and Elderly (60-80 years). The significance (p-value) of the differences between the three groups, computed using PERMANOVA (adonis) after considering the country-specific differences, is also indicated. The boxplots on the top show the variation of the top three PCoA coordinates for the samples belonging to the three age-groups. The elderly harboured a significantly different microbiome compared to the young/middle-aged.

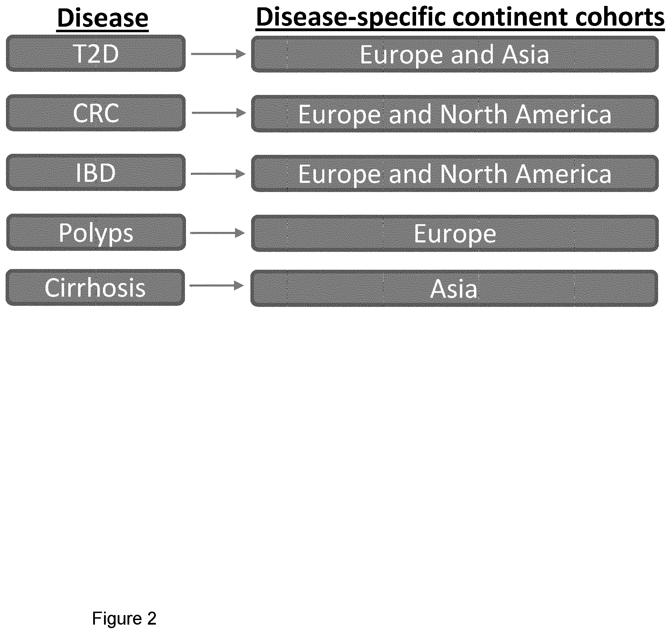

Figure 2. Disease specific continent-cohorts ensured geographical homogeneity across comparisons. The continent specific cohorts within which the analysis was restricted for each disease, to take into account the regional variations.

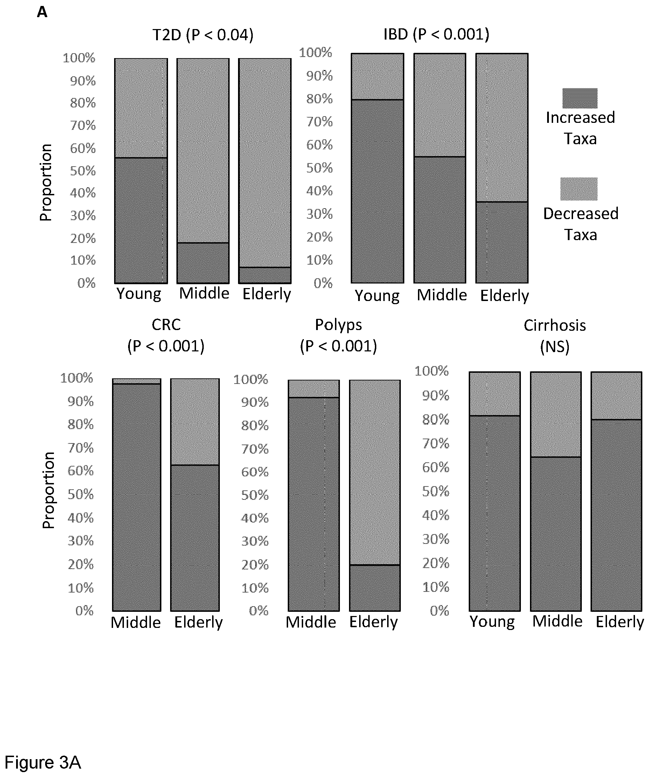

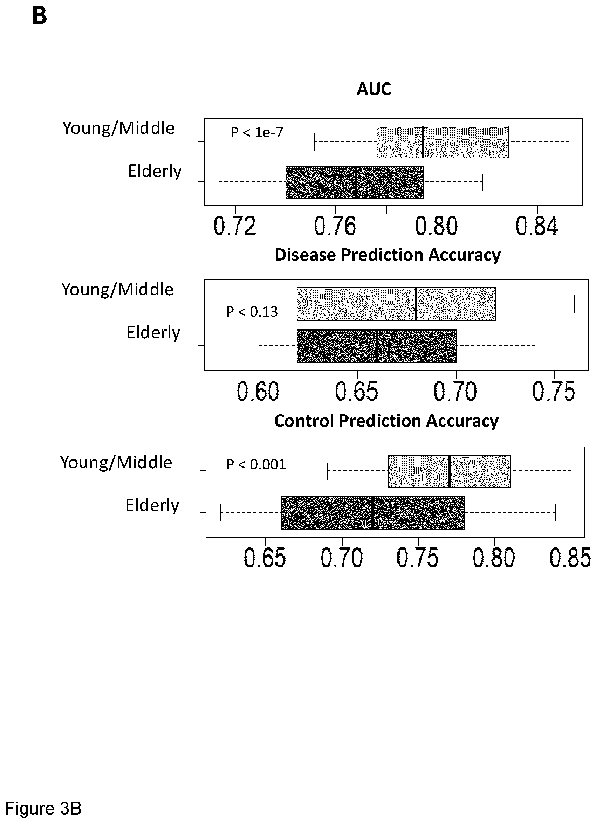

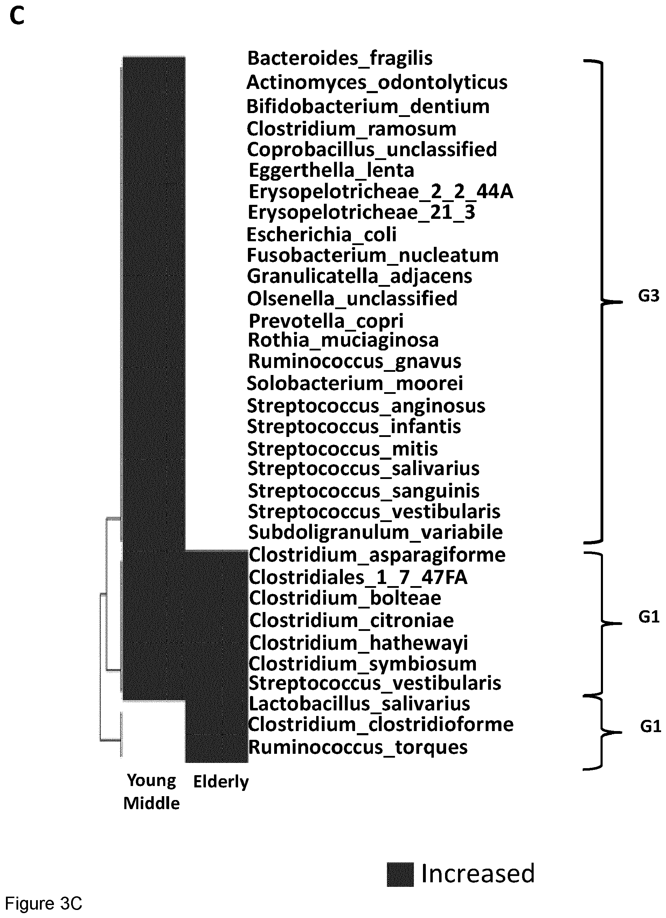

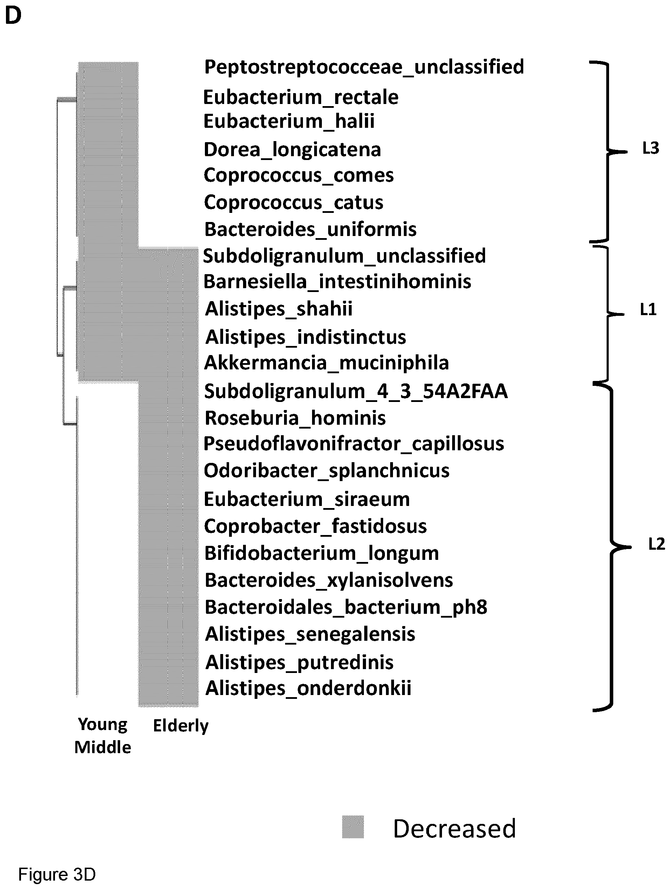

Figure 3. Age-related microbiome changes affect taxon abundance alterations for specific diseases, as well as the microbiome response shared by multiple diseases A. Comparison of the relative proportions of more abundant and less abundant disease-specific marker taxa across the young, middle-aged and elderly age-groups for the five diseases. For each disease-age-group scenario, we checked for the directionality (increased abundance in disease v/s decreased in disease) of association of the corresponding top disease-predictors by comparing their abundance trends in the control and diseased samples belonging to the specific age-groups (See Methods). To ensure that the results thus obtained were not affected by regional variations in microbiome composition, we again restricted these comparisons to the disease-specific continent cohorts. B. Comparison of the disease prediction AUCs, the disease classification sensitivity and control classification specificity of generic disease prediction models obtained for the elderly and young/middle-aged groups. Overall, the generic disease classifiers had a significant decrease in performance in the elderly age groups, indicating that shared microbiome response may be reduced in the elderly. Moreover, the loss of performance was especially significant with respect to the discrimination of control samples from disease. C and D. Heatmap of marker species showing consistent trends of either increase (C) or decrease (D) in at least two diseases in the elderly and young/middle-aged groups. The darker shading indicates consistent increase (C) in two or more diseases, the lighter shading indicates decrease (D) in two or more diseases. Based on their patterns of increase or decrease across the two age-groups, the taxa could be classified into six groups, namely G1-G3 and L1-L3.

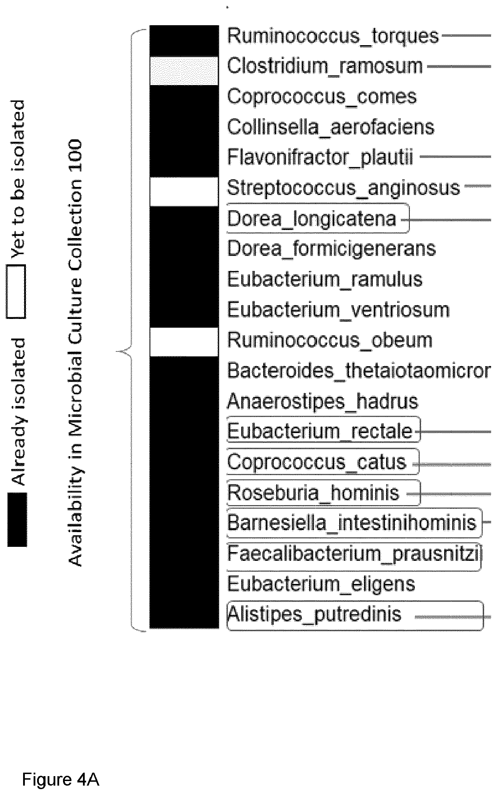

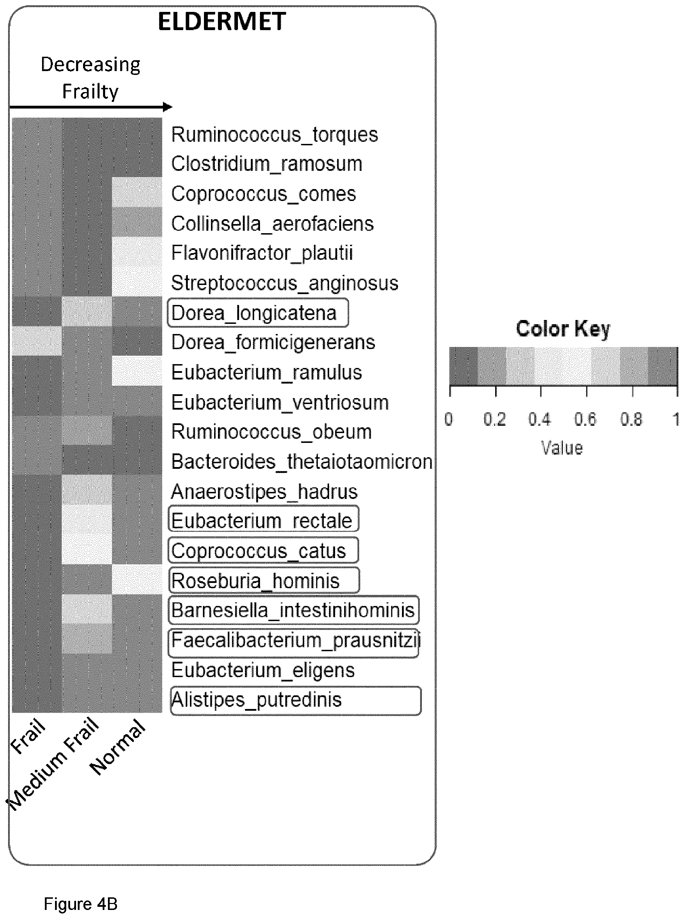

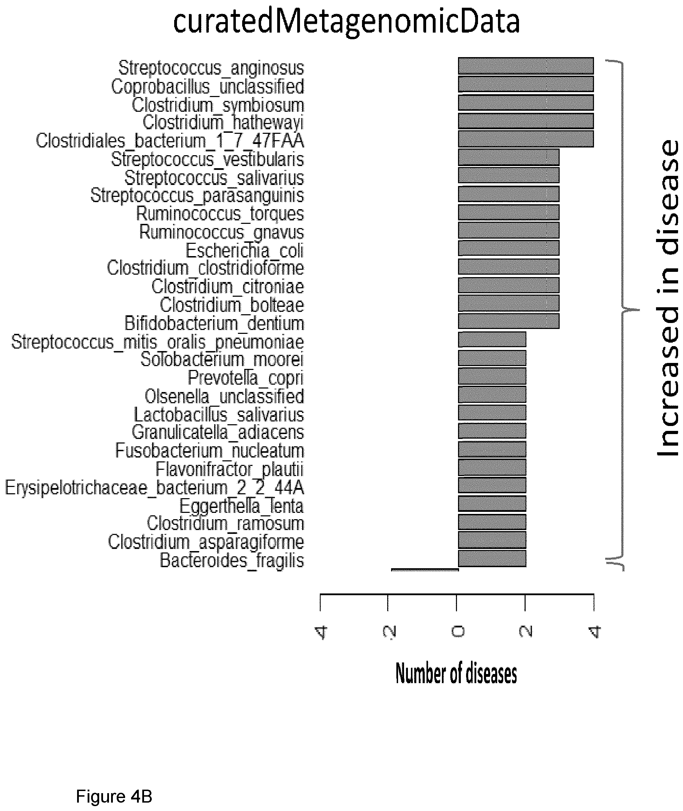

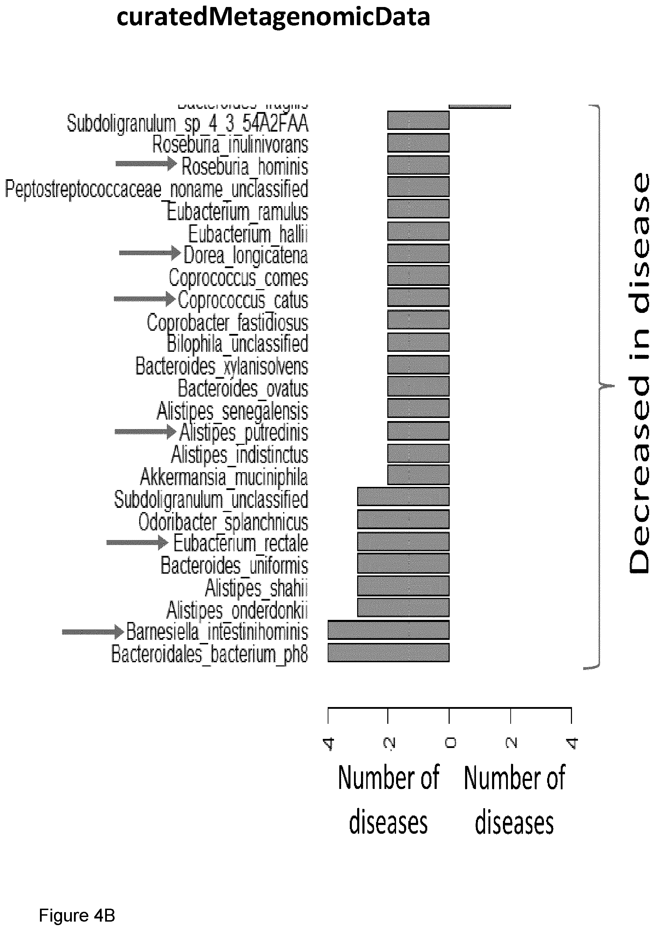

Figure 4. Identification of the seven strains for the probiotic consortium based on their reproducible association with reduced frailty and their loss in multiple diseases. A. Availability of the strains in the MCC100. B. The heatmap shows the ranked median abundance of some of the top frailty-predictive taxa (identified in the ELDERMET cohort) showing significant differential abundance across the three groups. Six of these taxa (highlighted in boxes) are observed to be decreased in multiple diseases (based on the meta-analysis in the curatedMetagenomicData). *Faecalibacterium prausnitzii,* although not detected in the curatedMetagenomicData meta-analysis has been associated with reduced frailty in the Twins-UK cohort.

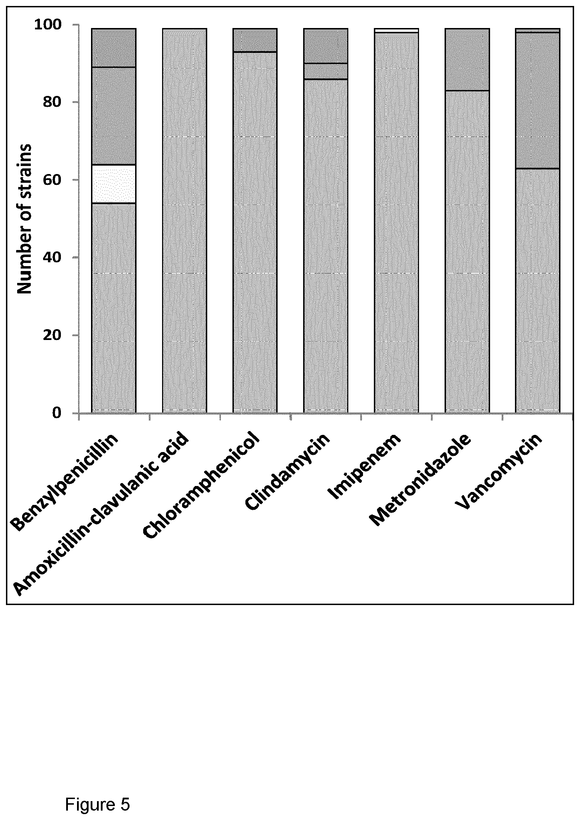

Figure 5 shows antimicrobial susceptibility of the MCC100 bacterial strains. The number of susceptible (bottom for all), intermediate resistant (second from bottom for Benzylpenicillin and top for Imipenem), resistant (top for Benzylpenicillin, Chloramphenicol, Clindamycin and Vancomycin) and naturally resistant (second from top for Benzylpenicillin, Clindamycin and Vancomycin and top for Metromidazole) strains is indicated for each antibiotic tested. Intrinsic resistance to Vancomycin was assumed for the non-tested strains for graphical representation.

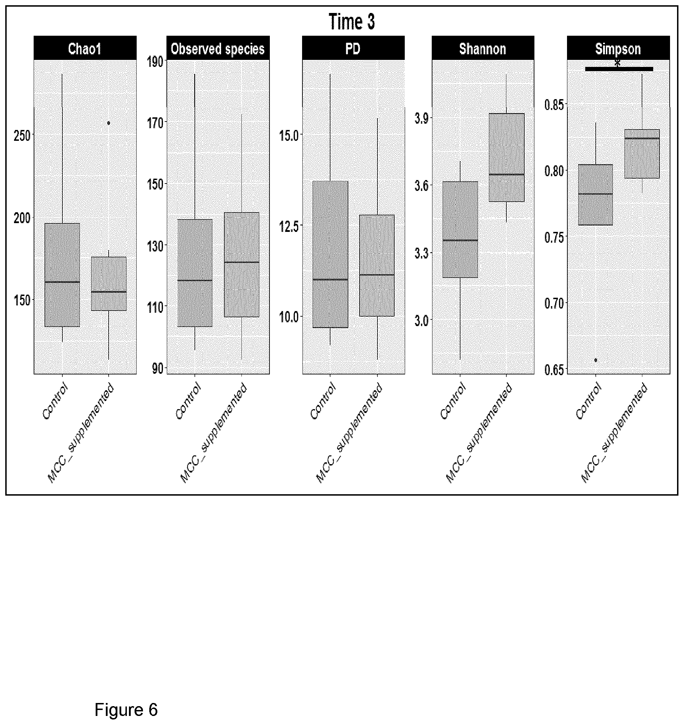

Figure 6 shows alpha-diversity indexes obtained from samples at the end of the fermentation run. Wilcoxon test performed for statistical analysis.

### Detailed Description of the Invention

The present inventors have developed the Microbiome Culture Collection 100 (MCC100), which is a general collection of putative probiotic microbial species that mimic the healthy gut microbiota composition and that could be used as a menu of single/consortium of next-generation probiotics for various indications, including not only aging and inflammation in the elderly, but also infectious diseases, inflammatory diseases, etc. The MCC100 is a panel of isolated and purified faecal microbiota species and strains that can be used in different combinations and proportions to prepare artificial consortia that can be used to rectify human diseases where alteration of the microbiota composition (dysbiosis) is implicated. The strains have been extensively characterised so that rational choices can be made among multiple isolates of the same species to deliver a consortium most appropriate for the particular application.

For any condition where dysbiosis is implicated, the faecal microbiota of a subject with the condition is compared with the normal faecal microbiota of healthy subjects. Any microbiota deficit or major difference is thus established and a modified artificial bacterial consortium that rectifies the dysbiosis is prepared by adjusting the composition and proportional abundance of bacterial strains in the original artificial bacterial consortium.

Frail older people have a gut microbiota composition characterised by an increase in *Bacteroidetes* and low alpha diversity compared to healthy older individuals. The present inventors have identified a set of bacterial taxa in the gut microbiome whose abundance corresponds inversely with microbiome disease response and frailty in older people and have defined a stable artificial microbiota consortium using an *in vitro* fermentation system to ultimately allow the rectification of the gut microbiota of frail elderly subjects using live biotherapeutic units with a defined microbial configuration. A bacterial consortium mimicking the healthy human gut microbiota may thus be provided to rectify changes in gut microbiota composition linked to increased frailty in older people.

In relation to sequences provided by the invention, sequence identity may be determined using a suitable mathematical algorithm. Computer implementations of such mathematical algorithms can be utilised for comparison of sequences to determine sequence identity. Such implementations include, but are not limited to, CLUSTAL in the PC/Gene program (available from Intelligenetics, Mountain View, California), the ALIGN program (Version 2.0) and GAP, BESTFIT, BLAST, FASTA and TFASTA in the Wisconsin Genetics Software Package, Version 8 (available from Genetics Computer Group (GCG), 575 Science Drive, Madison, Wisconsin, USA)). Suitably alignments using these programs may be performed using the default parameters.

As used herein, "sequence identity" or "identity" in the context of two nucleotide or polypeptide sequences makes reference to a specified percentage of residues in the two sequences that are identical when aligned for maximum correspondence over a specified comparison window, as measured by sequence comparison algorithms or by visual inspection. Suitably, a specified comparison window is selected from a sequence encoding or representing at least 20, at least 25, at least 30, at least 40, at least 50, at least 75, at least 80, at least 85, at least 90, at least 95, at least 100, at least 105, at least 110, at least 115, at least 120, at least 121, at least 122, at least 123, at least 150, at least 200, at least 250 or most preferably all of the amino acids of a specified polypeptide being aligned. In certain embodiments, the specified comparison window is all of the residues of the sequences. When percentage of sequence identity is used in reference to proteins it will be understood by those of skill in the art that residue positions which are not identical often differ by conservative amino acid substitutions, i.e. wherein amino acids are substituted with amino acids which have similar chemical properties to those amino acids which are replaced. The percent sequence identity may be adjusted upwards to correct for the conservative nature of a substitution.

Amino acids may be grouped according to the properties of their side chains, for examples as follows: (1) non-polar: Ala (A), Val (V), Leu (L), Ile (I), Pro (P), Phe (F), Trp (W), Met (M); (2) uncharged polar: Gly (G), Ser (S), Thr (T), Cys (C), Tyr (Y), Asn (N), Gln (Q); (3) acidic: Asp (D), Glu (E); and (4) basic: Lys (K), Arg (R), His(H). Alternatively, naturally occurring residues may be divided into groups based on common side-chain properties, for example: (1) hydrophobic: Norleucine, Met, Ala, Val, Leu, Ile; (2) neutral hydrophilic: Cys, Ser, Thr, Asn, Gln; (3) acidic: Asp, Glu; (4) basic: His, Lys, Arg; (5) residues that influence chain orientation: Gly, Pro; and (6) aromatic: Trp, Tyr, Phe. Conservative substitutions entail exchanging a member of one of these classes for another member of the same class, that is an amino acid residue with side chains having similar biochemical properties to the amino acid residue being substituted. Preferably when the amino acid sequences of the invention are modified by way of conservative substitution of any of the amino acid residues contained therein, these changes have no effect on the functional activity of the resulting polypeptide when compared to the unmodified polypeptide. The effect (if any) on function of a change in an amino acid residue may be easily assessed by a person skilled in the art, for example, by generating a mutation library, screening for changes in function and sequencing the mutants.

The following strains were deposited with NCIMB Ltd, Ferguson Building, Craibstone Estate, Bucksburn, Aberdeen, AB21 9YA, Scotland, UK by University College Cork - National University of Ireland (University College Cork), Room 447 Food Science Building, University College Cork, T12 K8AF, Cork, Ireland on 19 September 2019 and assigned accession numbers as follows: *Eubacterium rectale* (MCC552) deposited under NCIMB 43489, *Alistipes putredinis* (MCC001) deposited under NCIMB 43485, *Barnesiella intestinihominis* (MCC256) deposited under NCIMB 43486, *Roseburia hominis* (MCC694) deposited under NCIMB 43491, *Dorea longicatena* (MCC451) deposited under NCIMB 43488 and *Faecalibacterium prausnitzii* (MCC585) deposited under NCIMB 43490. *Coprococcus catus* (MCC394) was deposited with NCIMB Ltd, Ferguson Building, Craibstone Estate, Bucksburn, Aberdeen, AB21 9YA, Scotland, UK by University College Cork - National University of Ireland (University College Cork), Room 447 Food Science Building, University College Cork, T12 K8AF, Cork, Ireland on 3 October 2019 under NCIMB 43487. All strains were grown on YCFA broth.

It is understood that the strain of the present invention is not limited to the deposited strain since mutants and variants of the strain, for example, cell fusion strains or recombinant bacteria strains, may also be used in the invention. The term "mutant" is understood herein to refer to a microorganism which is derived from the deposited strain by one or more mutations. The mutant should retain the desired activity of the deposited strain or have improved activity over that of the deposited strain. These mutants can be related bacteria isolates that have either arisen spontaneously or under selection conditions designed to isolate the mutants. For example, commercial kits are available that generate random mutations following which all generated mutants can be screened for anti-microbial activity. The term "variant" is understood herein to refer to a microorganism which comprises the activity of the deposited strain. The variant should retain the desired activity of the deposited strain or have improved activity over that of the deposited strain. These variants can be related bacteria isolates that have either arisen spontaneously or under selection conditions designed to isolate the variants. The variants can also be recombinant bacteria which can be constructed using genetic engineering methods which would be well known to those skilled in the art. All generated variants may be easily screened for desired activity in subjects using frailty scores or biomarkers to measure age-related inflammation.

Treatment (i.e. the composition or the one or more strains) may be combined with one or more standard treatments or probiotics for the disease or condition in question. The treatment may be administered alone or may be administered as a pharmaceutical or probiotic composition which will generally comprise a suitable pharmaceutically acceptable excipient, diluent or carrier. The pharmaceutically acceptable excipient, diluent or carrier may be selected depending on the intended route of administration. Examples of suitable pharmaceutical carriers include water, glycerol and ethanol.

The treatment may be administered to a subject in need of treatment via any suitable route. In particular, the treatment may be administered orally or rectally. Routes of administration therefore include oral and rectal administration.

The treatment is typically administered to a subject in a "therapeutically effective amount", this being an amount sufficient to show benefit to the subject to whom the treatment is administered. The actual dose administered, and rate and time-course of administration, will depend on, and can be determined with due reference to, the nature and severity of the condition which is being treated, as well as factors such as the age, sex and weight of the subject being treated, as well as the route of administration. Further due consideration should be given to the properties of the treatment, for example, its *in-vivo* plasma life and concentration in the formulation, as well as the route, site and rate of delivery. Prescription of treatment, e.g. decisions on dosage, etc., is ultimately within the responsibility and at the discretion of general practitioners and other medical doctors, and typically takes account of the disorder to be treated, the condition of the individual patient, the site of delivery, the method of administration and other factors known to practitioners.

Dosage regimens can include a single administration, or multiple administrative doses. The treatment can further be administered simultaneously, sequentially or separately with other probiotics, therapeutics and medicaments which are used for the treatment of the disease or condition for which the treatment is being administered.

### Definitions

Unless otherwise defined, all technical and scientific terms used herein have the meaning commonly understood by a person who is skilled in the art in the field of the present invention.

The term "frailty" is used herein to describe a state of vulnerability to poor resolution of homeostasis following a stress and is a consequence of cumulative decline in multiple physiological systems over a lifespan.

"Faecal microbiota" as used herein is considered a to represent a non-invasive proxy for intestinal microbiota.

Typically, the terms "subject" and "patient" are used interchangeably herein. The subject is typically a mammal, more typically a human.

The term "treatment" as used herein and associated terms such as "treat" and "treating" means the reduction or inhibition of the progression, severity and/or onset of frailty or inflammation related to ageing (inflammaging), or at least one symptom thereof. The term "treatment" therefore refers to any regimen that can benefit a subject. Treatment may include curative, alleviative or prophylactic effects. In certain embodiments, the onset of frailty or inflammation related to aging is delayed.

A peptide, polypeptide, protein, bacteria or nucleic acid molecule of the invention may be provided in an "isolated" form. The term "isolated" as used herein means that the substance is provided in a form that differs from its naturally occurring environment. It may be used to refer to an *in vitro* preparation, isolation and/or purification of a peptide, polypeptide, protein, bacteria or nucleic acid molecule of the invention, such that it is not associated with *in vivo* substances or is substantially purified from *in vivo* substances or is present outside its naturally occurring environment.

In certain embodiments, the one or more bacterial species or strain is an isolated bacterial species or strain. An "isolated" bacteria is one which has been identified and separated and/or recovered from a component of its natural environment.

As used herein, a polypeptide can be considered to be "derived from" a strain if the polypeptide originates directly or indirectly from the strain. The polypeptide may be encoded by a part of the genome of the strain and may therefore be obtainable directly from culturing the strain, including for example being expressed in the strain, for example in microbial whole cells, being present in the cytosol thereof, being present in a cell culture thereof or being present in a cell lysate thereof. The polypeptide may also be synthetically prepared from a gene which is endogenous to the strain of the invention following isolation of the gene from the strain and sequencing of the gene. For example, the polypeptide may be synthetically prepared, and/or be obtained using recombinant DNA technology, such as from a genetically engineered plasmid/host cell system in which the plasmid includes a nucleic acid polymer which encodes the polypeptide.

As used herein the terms "nucleic acid" or "nucleotide sequence" includes genomic DNA, cDNA or RNA.

The phrase "consists essentially of" or "consisting essentially of" as used herein means that a polypeptide or nucleotide sequence may have additional features or elements beyond those described provided that such additional features or elements do not materially affect the desired function of the polypeptide. For example, a polypeptide consisting essentially of a specified sequence may contain one, two or three additional, deleted or substituted amino acids, at either end or at both ends of the sequence provided that these amino acids do not interfere with its function. Similarly, a polypeptide of the invention may be chemically modified with one or more functional groups provided that such functional groups do not interfere with its function.

The terms "polypeptide", "peptide", or "protein" are used interchangeably herein to designate a linear series of amino acid residues connected one to the other by peptide bonds between the alpha-amino and carboxy groups of adjacent residues. The amino acid residues are usually in the natural "L" isomeric form. However, residues in the "D" isomeric form can be substituted for any L-amino acid residue, as long as the desired functional property is retained by the polypeptide.

A peptide, polypeptide, protein, bacteria or nucleic acid molecule of the invention may be provided in a "purified" form. A nucleic acid or polypeptide of the presently disclosed subject matter is purified if it is substantially free of cellular material, viral material, or culture medium when produced by recombinant DNA techniques, or chemical precursors or other chemicals when chemically synthesised.

The words "comprises/comprising" and the words "having/including" when used herein with reference to the present invention are used to specify the presence of stated features, integers, steps or components, but do not preclude the presence or addition of one or more other features, integers, steps, components or groups thereof.

As used herein, terms such as "a", "an" and "the" include singular and plural referents unless the context clearly demands otherwise.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

### Example 1 - Preparation of original artificial bacterial consortium (MCC100)

Initially, a culture collection was achieved by using 25 different culture media to anaerobically isolate commensal microbes from the fresh faecal samples of seven healthy donors. A collection of 696 purified isolates was obtained and the species were identified by 16S rRNA gene sequencing analysis. The isolates belonged to 89 bacterial species from 39 different genera and 4 bacterial phyla, and one archaeon species. Recent major studies defining the core human gut microbiota composition including those that describe the elderly microbiota were reviewed, and the isolated taxa were classified as present or absent in the core microbiota. In order to reproduce the configuration of a healthy gut microbiota, and noting the taxa that are present in the core healthy gut microbiota, 73 commensal species belonging to 36 different genera were thus selected from the initial collection. 22 of the 73 species were singleton strains. For the others 51 species, RAPD-PCR analysis was performed in a subset of 171 isolates - up to 5 isolates per species from different donors were analysed when possible. At least two different primers were used with each isolate.

117 different profiles out of 171 isolates were obtained. In total, a collection of 139 different strains was thus identified. Finally, 100 different strains were selected, reflecting species with high abundance in a comparative analysis of the published core microbiome (Table 1). In order to maximize the genetic diversity of the consortium, strains were selected considering their lowest genetic similarity according to dendrograms that were constructed from the RAPD-PCR patterns. The defined consortium of 100 strains mimicking a healthy gut microbiota composition was termed the Microbiome Culture Collection 100 (MCC100). The inventors estimate that the consortium covers >90% of the most abundant species identified in the human faecal microbiota.

**Table 1. Genera selected for the consortium MCC100. The numbers of species and strains per genera are indicated. Taxonomic assignation performed by BLASTing whole genome sequences against filtered RDP database.**

| | **No. species** | **No. strains** |
|---|---|---|
| ***Phylum Firmicutes*** | **44** | **62** |
| *Anaerostipes* | 1 | 3 |
| *Blautia* | 3 | 5 |
| *Butyriciccocus* | 1 | 1 |
| *Catenibacterium* | 1 | 1 |
| *Clostridium sensu stricto* | 1 | 1 |
| *Clostridium XIVa* | 8 | 10 |
| *Clostridium XIVb* | 1 | 1 |
| *Coprococcus* | 3 | 5 |
| *Dorea* | 2 | 4 |
| *Enterococcus* | 2 | 3 |
| *Faecalibacterium* | 1 | 3 |
| *Flavonifractor* | 1 | 1 |
| *Fusicatenibacter* | 1 | 1 |
| *Gemmiger* | 1 | 1 |
| *Lachnospiracea Incertae Sedis* | 4 | 6 |
| *Lactobacillus* | 4 | 4 |
| *Oscillibacter* | 1 | 1 |
| *Robinsoniella* | 1 | 1 |
| *Roseburia* | 2 | 3 |
| *Ruminococcus* | 3 | 4 |
| *Streptococcus* | 1 | 2 |
| *Veillonella* | 1 | 1 |

| ***Phylum Bacteroidetes*** | 17 | 25 |
|---|---|---|
| *Alistipes* | 1 | 1 |
| *Bacteroides* | 11 | 18 |
| *Barnesiella* | 1 | 1 |
| *Odoribacter* | 1 | 1 |
| *Parabacteroides* | 2 | 3 |
| *Prevotella* | 1 | 1 |

| ***Phylum Proteobacteria*** | 4 | 5 |
|---|---|---|
| *Desulfovibrio* | 1 | 1 |
| *Escherichia*/*Shigella* | 2 | 3 |
| *Sutterella* | 1 | 1 |

| ***Phylum Actinobacteria*** | 4 | 7 |
|---|---|---|
| *Bifidobacterium* | 2 | 4 |
| *Collinsella* | 1 | 2 |
| *Propionibacterium* | 1 | 1 |

| ***Kingdom Euryarchaeota*** | 1 | 1 |
|---|---|---|
| *Methanobrevibacter* | 1 | 1 |

The whole genomes of the 100 strains were sequenced and the identity of the strains was validated by BLASTing the contigs against a filtered version of the RDP database with 16S genes sequences, confirming the 100 strains belonged to 70 different species (Table 1). 37 out of the 100 genomes sequenced are currently classified among the list of the Human Microbiome Project (HMP) "Most wanted" taxa for whole-genome sequencing priority. To further investigate the safety of the selected strains, their Minimum Inhibitory Concentration values for a panel of 7 antibiotics were determined using the E-test gradient strip method. Gram-negative bacteria (n=31) were tested for sensitivity to benzylpenicillin, amoxicillin-clavulanic acid, chloramphenicol, clindamycin, imipenem and metronidazole. Gram-positive bacteria (n=68) were also tested for vancomycin sensitivity. The Methanobrevibacter smithii MCC662 isolate could not be tested due to its inability to grow on plates. Amoxicillin-clavulanate inhibited the growth of all 99 bacterial strains (Figure 5). Imipenem inhibited 98 strains, with one strain showing intermediate resistance. Moreover, 93 strains were sensitive to chloramphenicol, 86 to clindamycin, 83 to metronidazole, 54 to benzylpenicillin and 63 out of 68 to vancomycin. Nevertheless, resistance to some antibiotics has been reported to be intrinsic in specific bacterial groups. Indeed, 48 strains had no resistance to any antimicrobial tested and 31 strains had endogenous resistance to up to 4 antibiotics, but this endogenous resistance presents a minimal risk for horizontal spread. All 99 of the bacterial strains could be inhibited by using amoxicillin-clavulanate or combinations of imipenem with any of the other antibiotics tested, or metronidazole with chloramphenicol.

### Example 2 - Gut microbiome alterations associated with increased frailty in the elderly

As described below, the identification of the key group of species involved two key steps (a. discovery and b. validation). The discovery step was to identify a group of species that decreased with increasing frailty in the elderly population. For this, the inventors focussed on the ELDERMET cohort of elderly Irish individuals (aged between 64-102 years of age). Shotgun faecal microbiome data for 215 individuals of this cohort was available, along with associated frailty indices and metabolomic profiles. This enabled the inventors to associate specific taxa with frailty changes. Using a combination of machine-learning and comparative statistics, the inventors identified a specific set of taxa that were predictive and demonstrated significant association with either increasing or decreasing frailty. While the identification of these specific taxonomic groups was significant, these associations could also be cohort-specific (i.e., Irish) or prevalent only within the elderly. Therefore, to validate their findings on a global scale (in the next validation step), the inventors utilised a second cohort of more than 2500 individuals belonging to multiple geographical locations (available in the curatedMetagenomicData repository of ExperimentHub database). While frailty status was not available for these individuals, there was data available on their disease status. The inventors hypothesised that if a species was therapeutic (prevented or delayed the onset of frailty), it should also have a high likelihood of being decreased in multiple diseases in this global cohort. Using region-homogenized machine learning based comparisons, the inventors identified a core-set of multi-disease markers that were either increased or decreased in multiple diseases in this global cohort. Importantly, a core group of six species overlapped between the two analyses. Other species like *Faecalibacterium prausnitzii,* which, although were not validated in the curatedMetagenomicData cohort, have been associated with reduced frailty in TwinsUK cohort. The inventors hypothesised that this core group of species, namely, *Coprococcus catus, Eubacterium rectale, Alistipes putredinis, Barnesiella intestinihominis, Roseburia hominis, Dorea longicatena* and *Faecalibacterium prausnitzii,* if administered as a therapeutic formulation, could prevent or delay the onset of frailty/disease in elderly subjects. Using a combination of analysis on multiple cohorts, the inventors thus identified this group of species for use as live biotherapeutics for both the physical and mental wellbeing of the elderly.

### MATERIALS AND METHODS

### Data collection from the curated Metagenomic Data repository

Since the focus of investigation was the gut microbiome, a subset of the curated Metagenomic Data, containing 4,195 stool samples was selected (annotated as 'body_site': stool). Samples which did not have defined age and study-condition were removed, thereby filtering the dataset to 3,580 samples. From this set, samples having age less than 20 years of age were further removed (retaining 2,564 samples). Notwithstanding the uniform bioinformatics analysis strategy applied to this data, two major factors that may contribute an artefactual bias in multi-cohort microbiome datasets (and which were available in the metadata) are the read-length (obtained from the sequencer) and DNA extraction methodologies (which are study-specific). To test the effect of these factors, samples from Peruvian, African and Fijian individuals were first removed in order to remove the confounding effects of region/life-style-specific, along with those from hospitalized individuals. Subsequently, on the remaining subset, we evaluated the effect of these factors using envfit on the species level profiles by first visually comparing the differences using PCoA and then testing the confidence of these differences using envfit. For this purpose, we performed 20 boot-strapped envfit iterations, each time taking a subset of samples (sub-sample size: 200) and computing the R² and the significance (P-value) of the differences, and then comparing the distribution obtained with that obtained using a null distribution obtained by taking 200 sub-samples (after permuting the labels). We established that while read lengths had a marginal effect (R=9e-3, P < 0.06) (Figure 1), samples from one of the studies (Schirmer, M., Smeekens, S. P., Vlamakis, H., Jaeger, M., Oosting, M., Franzosa, E. A., et al. (2016). Linking the Human Gut Microbiome to Inflammatory Cytokine Production Capacity. Cell, 167(4), 1125-1136 e1128. doi:10.1016/j.cell.2016.10.020), using a DNA extraction technique tagged as 'Illuminakit' in the metadata, had a distinct taxonomic profile. We removed the 465 samples of this study from all further analyses, thereby reducing the effect of extraction methodology on taxonomic profiles (P < 0.09; Figure 1). To this compiled list, we added the samples from four recently published datasets (one IBD-specific dataset of 220 samples, referred to as 'FranzosaEA_2018' (Franzosa, E. A., Sirota-Madi, A., Avila-Pacheco, J., Fornelos, N., Haiser, H. J., Reinker, S., et al. (2019). Gut microbiome structure and metabolic activity in inflammatory bowel disease. Nat Microbiol, 4(2), 293-305. doi:10.1038/s41564-018-0306-4)); three CRC-Specific datasets referred to as 'WirbeIJ_2019', ThomasAJ_Cohort1' and ThomasAJ_Cohort2' (Thomas, A. M., Manghi, P., Asnicar, F., Pasolli, E., Armanini, F., Zolfo, M., et al. (2019). Metagenomic analysis of colorectal cancer datasets identifies cross-cohort microbial diagnostic signatures and a link with choline degradation. Nat Med. doi:10.1038/s41591-019-0405-7; Wirbel, J., Pyl, P. T., Kartal, E., Zych, K., Kashani, A., Milanese, A., et al. (2019). Meta-analysis of fecal metagenomes reveals global microbial signatures that are specific for colorectal cancer. Nat Med. doi:10.1038/s41591-019-0406-6). This repository, along with the 189 shotgun sequenced samples from the ELDERMET cohort, resulted in a total of 2,564 samples.

### Effect of host-associated factors, including age groups on the microbiome profile

Some of the investigated metadata were observed to be redundant and had similar associations (examples included groups like Country and Dataset Name; Age and Age-category; Study condition and Disease; Antibiotics current use and Antibiotics family). In these cases, we retained the former metadata and removed the latter ones. We added another region-specific metadata, namely Continent, for reasons explained in the subsequent section. Subsequently, we filtered out those metadata present in less than 30% of the samples. A total of six metadata remained. We obtained the association of each of these metadata using PERMANOVA (using the adonis function of the vegan R package).

For investigating the variation of the microbiome with age across the adult-hood landscape, the individuals were binned into three age groups namely young (20-40 years of age), middle-aged (40-60 years) and elderly (60-80 years). We removed the antibiotic-treated subjects from all subsequent analyses. Principal component analysis of the microbiome profiles of the samples belonging to the three age-groups was performed and plotted using dudi.pco and s.class function of the ade4 R package. The significance of the association was obtained using the PERMANOVA (adonis function) implemented in the 'vegan' R package (with 'country' as a confounder).

### Grouping samples into continent-specific bins

Given that regional factors had the highest effect on the microbiome composition, it was important to ensure regional homogeneity for comparative disease-association analysis. However, the majority of disease-specific cohorts either displayed significant differences in age difference in the age of the control and diseased individuals (i.e. they were not age matched) or biases for disease patients from specific age-groups (Figure 1A). Grouping the samples into continent-level bins would address the issue of limited sample numbers for various diseases across the age-groups, whilst maintaining regional homogeneity of the cohorts (Figure 1A). To compare the overall effects of the two regional factors, country and continent, on the microbiome profiles we performed bootstrapped PERMANOVAs (by taking 20% subsets) within the control individuals. The results indicated that, although continent was observed to have a marginally lower effect on the microbiome composition compared to nationality (country), performing repeated bootstrapped comparisons indicated the effect of continent to have a higher significance (calculated as -log of Adonis P-values) than the country on the microbiome profiles (Figure 1A). For each disease, the continent specific affiliations of the disease cohorts were first obtained. Subsequently, we performed all subsequent performing the investigations pertaining to each disease by pooling samples belonging to the same continent as the corresponding disease cohorts. This was expected to optimally homogenize the region-specific variations, while ensuring enough representation of various diseases (and controls) across age-groups.

### Disease classification using randomForest

For each disease-age group combination (that is identifying the disease-associated markers in a specific age-group), we performed 100 iterations, such that in each iteration, we trained the classifier on a subset of disease and the same number of control samples (denoted as 'training subset', n=20) (belonging to an age-group). The classifier performance (in terms of the sensitivity, specificity and AUC) was subsequently tested on a subset of the remaining samples within that age-group, as well as on randomly selected equally sized subsets of samples (denoted as 'testing subsets'). For a given disease, to ensure that the observed changes were not artefactual consequences of differences in sizes of training and testing subsets (for each of the testing age-groups, n=20), we kept the training and testing subset sizes constant across all training age-groups. The classification AUCs, Specificities and Sensitivities were computed using the various modules in the pROC package.

### Identifying the top disease-associated markers for the different age-groups

For each disease-age group scenario (as described above), we ranked the species-level taxa in decreasing order of their importance scores (mean decrease in GINI). The taxa having importance scores in the top 15 percentile (that is higher than 85 percentile) were identified as the top 85 percentile predictors/markers for a given disease in that age-group. We then focussed on the species detected as being within the set of the top 85-percentile markers across at least one age-group.

### Determining the directionality of disease association for the top 85 percentile markers in each disease-age group scenario

To obtain the directionality (increased or decreased in disease) of the top 85 percentile markers corresponding to a disease in an age-group, Mann-Whitney U tests were performed to compare their abundances in the control and diseased samples from the specific age-group. To further ensure that the results thus obtained were not affected by regional variations in microbiome composition, we restricted these comparisons to the disease-specific continent cohorts. Nominal P-values obtained after the Mann-Whitney U tests were adjusted using Benjamini-Hochberg correction. The top markers having significant change in their abundance with FDR corrected P-values < 0.05 were then filtered (Figure 3). The directionality of these markers was assigned based on the trends of their abundance patterns, as either 'Increased' or 'Decreased' in disease.

### Creation of generic disease prediction models and identification of shared disease markers

The generic disease prediction classifiers were developed in a manner similar to that shown in Figure 1. The only difference was the agglomeration of equal number (n= 10) samples from each of the diseases, rather those of a specific disease as described below. For each age-group (young/middle-aged or elderly), a specific disease cohort was created by taking equally size sub-samples from each disease (to remove biases in the classifiers originating from specific diseases). The sub-sample size was also kept same across the age-groups to ensure uniformity in the testing and training sizes of the classifiers across all age-groups. The iteration was subsequently repeated five times using a different (but equally sized) subset of diseased and control samples (as described above). The AUC and sensitivities for the five repetitions were then merged and compared across the young/middle-aged and elderly. To remove regional biases in microbiome compositions affecting these results, all analyses were restricted within the disease-specific continent cohorts (Figure 1).

### Identification of Frailty-associated markers

We used a random forest model to regress both the Functional Independence Measure (FIM) and the Barthel Score (both an inverse measure of frailty) of an individual from the microbiome profile. Random forest regression training was performed on 20% of the samples and tested on the remaining 80%. The ranked feature importance scores of the different species were then obtained. Microbiome features (that is the species) were ranked in decreasing order of their feature importance scores (mean decrease in GINI coefficient upon excluding the feature). We subsequently divided the individuals into three equal tertiles, frail, medium frail and normal, based on their FIM values. Within these three groups, for the top 100 markers thus detected, we performed comparisons (using Kruskal-wallis H test, post-hoc using dunns' test) to identify which of the markers exhibited significant differences across the three groups. We then compared the overlap of these markers with the disease-associated markers shared across multiple diseases in at least one of the three age-groups.

### Creating metabolite species maps and obtaining the metabolic signature of a given group of species

We utilised the literature-curated experimentally annotated species to metabolite (production/consumption) associations available as part of the Virtual Metabolic Human database as well as those obtained in a recent meta-analysis by Sung *et* al (Noronha, A., Modamio, J., Jarosz, Y., Guerard, E., Sompairac, N., Preciat, G., et al. (2018). The Virtual Metabolic Human database: integrating human and gut microbiome metabolism with nutrition and disease. Nucleic Acids Res. doi:10.1093/nar/gky992; Sung, J., Kim, S., Cabatbat, J. J. T., Jang, S., Jin, Y. S., Jung, G. Y., et al. (2017). Global metabolic interaction network of the human gut microbiota for context-specific community-scale analysis. Nat Commun, 8, 15393. doi:10.1038/ncomms15393), to create a species-to-metabolite map of more than 300 metabolite production and consumption profile corresponding to 992 species in a 0 (absent) and 1 (present) notation. For each microbiome, the metabolite production/consumption capability was then obtained as the matrix inner product of the abundance profile of the species and the species-to-metabolite map thus obtained.

To identify metabolite profiles significantly associated with a group, the number of species in this group harbouring each metabolite was first obtained and compared with that obtained for the other species not present in this marker list using Fishers' exact test. The four values, namely the number of species in the marker group harbouring a metabolite profile, the number of species in the marker group not harbouring the profile, the number of other species harbouring the metabolite profile and the number of other species not harbouring the metabolite profile were input as a contingency-matrix to the fisher.test function of R. Markers with fold change greater than 1 and nominal p < 0.05. A Benjamini-Hochberg correction of p-values was then performed on this subset using p.adjust function of R. Metabolite profiles with corrected p-values of less than 0.1 were finally identified. The above approach was also applied for the shared gain (G1-G3) and loss (L1-L3) groups.

### RESULTS

### Identification of disease-associated microbiome markers

A stepwise methodology was adopted to reduce the confounding effects of DNA sequencing/extraction methodologies on the microbiome profiles from the different studies (See Methods; Figure 1), and subsequently retained samples from individuals with age in the range of 20-89 years (excluding cohorts where the "controls" also consisted of hospitalised patients). This data set was supplemented with 475 shotgun metagenome profiles from recently published studies on IBD (Franzosa et al., 2019) and CRC (as "Validation" cohorts) (Thomas et al., 2019; Wirbel et al., 2019), finally assembling a collated set of microbiome taxonomic profiles from more than 2,500 samples (including the 189 ELDERMET samples used later in the study).

The interaction of metadata with taxonomic profiles was next investigated. After filtering out redundant and sparse (recorded for less than 30% of the samples) metadata types (See Methods), regional factors namely country and continent had the largest interaction with gut microbiome composition (Figure 1A). Regional factors reflect the ethnicity and other socio-economic properties of the study populations, which has a dominant effect on gut microbiome architecture and microbiome-based disease signatures. Age and study-condition (disease versus control status) were the second major effect. The variation of the apparently "healthy" microbiome across the age landscape was explored using Principal Component Analysis of 1,175 gut microbiome profiles from exclusively "control" individuals from the age-groups '20-40' (categorized as "Young"), "40-60" ("Middle-Age") and "60-80" ("Elderly"). The elderly controls had a distinct microbiome composition compared to the young and the middle-aged (Figure 1B), in line with previous findings.

Given that regional factors like country or continent had the highest effect on the gut microbiome composition, any investigation into disease-specific microbiome signatures for the various diseases required the comparisons to be performed within geographically homogeneous sub-populations (or study cohorts) to ensure that any differences in disease signatures were not simply driven by regional variations in gut microbiome composition.

This issue was addressed by grouping the samples into continent-level cohorts. This ensured sufficient representation of samples from the three different age-groups for all diseases (Figure 2).

Subjects were divided (in the continent-specific cohorts corresponding to each disease) into three 20-year age bands (as described above) and 100 iterations performed, each time training Random Forest classifiers on a subset of samples belonging to an age band, and evaluating the disease classification performance on the samples from the same age band (excluding the samples used for training) or the different age-bands.

For each disease, the age-group specific feature importance scores for each species was computed and the species having marker scores in the top 85 percentile individually for each of the age-groups was identified.

### Age-specific changes in the directionality of taxon abundance alterations for specific diseases, and overlap of taxa in the species-level microbiome response shared by multiple diseases

We next investigated if the diseases were characterised by distinct patterns of microbial taxon gain or loss, even across the different age-groups. For each disease-age-group scenario, we determined for the directionality (increased versus decreased in disease) of association of the corresponding top 85-percentile disease-predictors by comparing their abundance trends in 'region-matched' control and diseased samples (See Methods). Across the age-groups, for four of the five diseases (i.e. not cirrhosis), there was a change in the directionality of the microbiome alteration, characterised by a significant reduction in the number of gained features with older age (Figure 3A). Thus, the microbiome alterations in most of these diseases are characterised by a gradual shift from a state dominated by gained microbiome components to an increasing loss of control-associated taxa in the elderly. The above results highlight that the loss of specific beneficial taxa is more important for disease onset in elderly individuals than gain of pathobionts. It further highlights that, for the elderly, microbiome-restoration strategies may be important for maintaining a positive health status.

The overlap of altered taxa across diseases reported in one study was 51%, while another study reported that generic control versus disease classifiers trained by agglomerating disease samples from multiple studies could still distinguish controls from disease with an AUC of greater than 0.8. We could identify specific groups of species that displayed consistent trends of associations with multiple diseases only within certain age-groups. The first group had elderly-specific associations with multiple diseases and included *Barnesiella intestinihominis, Collinsella aerofaciens, Bifidobacterium longum* (each detected across four diseases), *Alistipes senegalensis, Anaerostipes hadrus, Clostridium leptum, Alistipes indistinctus* and *Eubacterium ramulus.* The second group, including *Streptococcus salivarius* and *Ruminococcus gnavus,* displayed multiple disease associations, only within the young/middle-aged. Given that microbiome composition changes with age, we investigated the effect of age on the extent of these shared disease responses. We designed generic disease classifiers (using Random Forest), taking equally sized sub-samples of controls and diseased individuals (containing equal number of samples from each disease to prevent disease/age-group specific biases in classification performance (Figure 3). While the performances of the generic disease prediction models in the young/middle-aged was high (median AUC: 0.79) and similar to those reported by earlier studies, the same models applied to data from elderly subjects had significantly lower performance AUC (P < 1e-7) (Figure 3B). Moreover, in these models, while no significant differences were observed with respect to the disease prediction sensitivities (P < 0.13), the specificity of prediction (that is the accuracy of identifying healthy individuals) was significantly lower for the elderly. This was not an effect of the differential representation of samples from the different diseases, as we had ensured equal representation of all diseases across all age-groups. Thus, in contrast to previous meta-analyses, the shared disease response was significantly lower across elderly subjects, primarily with respect to the discrimination of non-diseased individuals. It indicates that with ageing, the microbiome differences that emerge between the healthy and the diseased subjects become progressively weaker, further re-iterating that the retention of health-associated species is more important for the elderly. Furthermore, our clarification of the effect of age on disease-associated taxa provides a refined set of features for improved microbiome-based diagnostics for these diseases.

Next, we sought to characterise the elements of the shared disease response. Notably, closer inspection of the directionality of the associations indicated specific taxa with consistent trends of association with multiple diseases (based on the trend shown in Figure 3). The overall patterns of taxon gain or loss encompassed several trends observed by earlier studies. For example, *Streptococcus anginosus* and *Fusobacterium nucleatum* were detected as gained in multiple diseases. Similarly, species belonging to Roseburia spp. (R. *hominis*) were lost. We identified a total of 57 species that showed consistent directionality of association with multiple diseases in either young/middle-aged or the elderly age-groups (Figure 3C and 3D). Based on their differential detection profiles in the shared response across age-groups, we assigned these into six different groups, namely G1 (increased in disease across all age groups), G2 (increased in disease only in the elderly), G3 (increased only in young/middle-aged), L1 (decreased in disease across both), L2 (decreased only in the elderly) and L3 (decreased only in the young/middle-aged groups) (Figure 3C and 3D). Many of the species previously reported as associated with shared gain or loss across multiple diseases belonged to the G3 group, that is, they showed similar trends of gain or loss in disease (as reported earlier) in the young and the middle-aged groups, but not in the elderly. These included the *Streptococci, Fusobacterium nucleatum, Escherichia coli* and *Bacteroides fragilis.* In contrast, a separate group of species including *Ruminococcus torques, Clostridium clostridioforme* and *Lactobacillus salivarius* were associated with multiple diseases only in the elderly. Finally, we identified a distinct group of species (G1) that was gained across diseases in both elderly and young/middle aged groups. These included a group of Clostridia (*C*. *bolteae, C. symbiosum, C. hathewayi, C. citronae, C. asparagiforme*) (Figure 3C). These taxa have been identified in separate studies of different diseases and/or disease-like states, but are shown here, for the first time to be part of a shared gain response across diseases. Based on these findings, we hypothesise that this specific G1 group of species constitute a shared disease response associated with a general patho-physiological failure in the affected individual.

### Reproducible association of the G1 disease-positive markers with increased frailty in elderly individuals from the ELDERMET cohort

Frailty in the elderly is characterised by reduced function of multiple systems. We investigated if the taxon associations with frailty could be experimentally validated in the ELDERMET cohort, for whom we had both shotgun metagenome and faecal metabolomic data. Using Random Forest regression (with five-fold cross validation), we could predict the frailty of an individual (testing both community- and residential care-dwelling subjects) based on frailty-associated taxa. Subsequently, we identified the top 100 markers with the best predictive ability and compared their abundances across the frail, medium frail and normal (apparently healthy) individuals and identified a subset of markers that showed significant decrease in the frail group of individuals (Figure 4; See Methods). Interestingly, a subset of 6 of these taxa were also observed to be reproducibly associated with a loss in multiple diseases in the curatedMetagenomicData. It was this group of taxa that were identified to be our markers for amelioration of frailty.

### Discussion

Figure 4 shows that certain species associated with either increased or reduced frailty in the ELDERMET cohort are also observed to be respectively increased or decreased in multiple diseases in the samples constituting the curatedMetagenomicData repository. Wilcoxon test were performed for statistical analysis. Given their reproducible association with reduced frailty and negative association with multiple diseases, we hypothesise that the group of organisms detailed herein, when administered either together or in specific combinations as a live biotherapeutic consortium, have the potential to retain microbiome resilience and prevent or delay onset of multiple diseases and frailty.

### Example 3 - Use of strains from MCC100 to increase alpha-diversity in an in vitro human colonic model

The capacity of the MCC100 to modulate elderly microbiota types was tested in an *in vitro* colon model. The fermentation system was inoculated with faecal samples from elderly donors - 3 healthy and 3 frail elderly microbiota types were used. Each sample was run with the MCC100 supplementation or without (control) in duplicate. The system was run for 3 days in continuous flow at 37°C and pH 6.8. The microbiota composition of samples collected at time 0 (just after inoculation) and time 3 (after 3 days of culture) was analysed by 16S rRNA gene sequencing. Analysis of the time 0 samples showed the supplementation of the faecal samples with the MCC100 resulted in the expected increase of several alpha-diversity indexes, Chao1, Observed species, Shannon and Simpson being significantly different. After 3 days of culture and as a consequence of the microbial adaptation to the fermenter conditions, the alpha-diversity drops compared with time 0 values. However, samples supplemented with the MCC100 showed greater Shannon and Simpson indexes values than the control group, Simpson index being significantly different (Figure 6). This outcome supports the hypothesis that administration of the MCC100, or a subset thereof, could add unique taxa or modify the abundances of the populations in complex communities (such is in humans). The analysis of the fermenter data by microbiota types indicates that the MCC100 was able to increase the alpha-diversity after 3 days of culture in both healthy and frail microbiota profiles, as indicated by the numerical rise of Shannon and Simpson indexes in the MCC100 supplemented groups compared with control groups. Alpha-diversity of frail samples also showed higher values for the indexes Chao1, Observed species and PD when MCC100 was added. Despite the indexes showing the same trend, the differences were not statistically significant, likely due to low power of the sample size. Furthermore, analysis of the microbial Beta-diversity by Unweighted UniFrac showed that frail samples supplemented with MCC100 mapped closer to the healthy samples than frail control samples after fermentation culture.

Modulation of the abundance of some well characterised, health-related taxa by MCC100 addition was observed at the end of the fermenter run. Indeed, an increase in the population of the health-related Faecalibacterium genus was reported, as well as increases in Sutterella and Escherichia/Shigella. The frailty-related bacterium Anaerotruncus showed a slight reduction and Clostridium XIVa abundance was also decreased. Comparison of unique and shared taxa between MCC100-supplemented and control samples indicated that the addition of MCC100 promoted maintenance of a greater number of species in both healthy and frail sample groups in this artificial system. Since MCC100 is a customizable consortium, different designs could be developed to optimise the modulation of taxa in different states of microbiota alteration (so-called dysbiosis).

In conclusion, a collection of commensal gut microbial isolates has been established by rationally selecting a consortium of 100 strains and defining their genetic relatedness, antibiotic susceptibility and genome coding capacity. The results indicate that the MCC100, or a subset thereof, could be used as a live biotherapeutic product since it modulates the gut microbiota of elderly donors by increasing the alpha-diversity in the *in vitro* human colonic model.

## Claims

1. A composition comprising at least two isolated bacterial species or comprising a purified bacterial preparation comprising at least two bacterial species, wherein the at least two bacterial species are selected from the group consisting of *Coprococcus catus, Eubacterium rectale, Alistipes putredinis, Barnesiella intestinihominis, Roseburia hominis, Dorea longicatena* and *Faecalibacterium prausnitzii.*

2. The composition as claimed in claim 1, wherein the at least two bacterial species are selected from the group consisting of *Coprococcus catus* comprising amino sequence SEQ ID No. 1 or a variant sequence having at least 90% sequence identity thereto, *Eubacterium rectale* comprising amino sequence SEQ ID No. 2 or a variant sequence having at least 90% sequence identity thereto, *Alistipes putredinis* comprising amino sequence SEQ ID No. 3 or a variant sequence having at least 90% sequence identity thereto, *Barnesiella intestinihominis* comprising amino sequence SEQ ID No. 4 or a variant sequence having at least 90% sequence identity thereto, *Roseburia hominis* comprising amino sequence SEQ ID No. 5 or a variant sequence having at least 90% sequence identity thereto, *Dorea longicatena* comprising amino sequence SEQ ID No. 6 or a variant sequence having at least 90% sequence identity thereto and *Faecalibacterium prausnitzii* comprising amino sequence SEQ ID No. 7 or a variant sequence having at least 90% sequence identity thereto.

3. The composition as claimed in claim 2, wherein the at least two bacterial species are selected from the group consisting of *Coprococcus catus* comprising amino sequence SEQ ID No. 1, *Eubacterium rectale* comprising amino sequence SEQ ID No. 2, *Alistipes putredinis* comprising amino sequence SEQ ID No. 3, *Barnesiella intestinihominis* comprising amino sequence SEQ ID No. 4, *Roseburia hominis* comprising amino sequence SEQ ID No. 5, *Dorea longicatena* comprising amino sequence SEQ ID No. 6 and *Faecalibacterium prausnitzii* comprising amino sequence SEQ ID No. 7.

4. The composition as claimed in any one of claims 1 to 3, wherein the at least two bacterial species are selected from the group consisting of *Coprococcus catus* (MCC394) deposited under NCIMB 43487, *Eubacterium rectale* (MCC552) deposited under NCIMB 43489, *Alistipes putredinis* (MCC001) deposited under NCIMB 43485, *Barnesiella intestinihominis* (MCC256) deposited under NCIMB 43486, *Roseburia hominis* (MCC694) deposited under NCIMB 43491, *Dorea longicatena* (MCC451) deposited under NCIMB 43488 and *Faecalibacterium prausnitzii* (MCC585) deposited under NCIMB 43490.

5. The composition as claimed in any one of claims 1 to 4, wherein the composition comprises *Coprococcus catus, Eubacterium rectale, Alistipes putredinis, Barnesiella intestinihominis, Roseburia hominis, Dorea longicatena* and *Faecalibacterium prausnitzii.*

6. A pharmaceutical or probiotic composition comprising a composition as claimed in any one of claims 1 to 5 and a pharmaceutically acceptable carrier.

7. A composition as claimed in any one of claims 1 to 5 for use as a medicament.

8. A composition as claimed in any one of claims 1 to 5 for use in treating or delaying onset of frailty in a subject in need thereof.

9. A composition as claimed in any one of claims 1 to 5 for use in treating or delaying onset of inflammation related to aging in a subject in need thereof.

10. An isolated bacterial strain selected from the group consisting of *Coprococcus catus* (MCC394) deposited under NCIMB 43487, *Eubacterium rectale* (MCC552) deposited under NCIMB 43489, *Alistipes putredinis* (MCC001) deposited under NCIMB 43485, *Barnesiella intestinihominis* (MCC256) deposited under NCIMB 43486, *Roseburia hominis* (MCC694) deposited under NCIMB 43491, *Dorea longicatena* (MCC451) deposited under NCIMB 43488 and *Faecalibacterium prausnitzii* (MCC585) deposited under NCIMB 43490.

11. An isolated bacterial strain selected from the group consisting of *Coprococcus catus* comprising amino sequence SEQ ID No. 1, *Eubacterium rectale* comprising amino sequence SEQ ID No. 2, *Alistipes putredinis* comprising amino sequence SEQ ID No. 3, *Barnesiella intestinihominis* comprising amino sequence SEQ ID No. 4, *Roseburia hominis* comprising amino sequence SEQ ID No. 5, *Dorea longicatena* comprising amino sequence SEQ ID No. 6 and *Faecalibacterium prausnitzii* comprising amino sequence SEQ ID No. 7.

12. An isolated bacterial strain selected from the group consisting of *Coprococcus catus* comprising a 16S rDNA comprising SEQ ID No. 15 or a sequence at least 99.5% identical thereto, *Eubacterium rectale* comprising a 16S rDNA comprising SEQ ID No. 16 or a sequence at least 99.5% identical thereto, *Alistipes putredinis* comprising a 16S rDNA comprising SEQ ID No. 17, *Barnesiella intestinihominis* comprising a 16S rDNA comprising SEQ ID No. 18 or a sequence at least 99% identical thereto, *Roseburia hominis* comprising a 16S rDNA comprising SEQ ID No. 19, *Dorea longicatena* comprising a 16S rDNA comprising SEQ ID No. 20 and *Faecalibacterium prausnitzii* comprsing a 16S rDNA comprising SEQ ID No. 21 or a sequence at least 99.5% identical thereto.

13. A method of preparing a modified artificial bacterial consortium for use in treating a condition where dysbiosis is implicated, the method comprising:
- comparing normal faecal microbiota of healthy subjects with faecal microbiota of a subject to be treated to identify alterations in the microbiota of the subject with the condition; and
- adjusting the composition and proportional abundance of bacterial strains in an original artificial bacterial consortium to provide a modified artificial bacterial consortium that rectifies the identified alterations.

14. The method as claimed in claim 13, wherein the original artificial bacterial consortium comprises the genera shown in Table 1.

15. The method as claimed in claim 13 or 14, wherein the condition where dysbiosis is implicated is frailty.
